# EUROPÄISCHE PATENTSCHRIFT

(11) **EP 2 111 824 B1**
(45) Veröffentlichungstag und Bekanntmachung des Hinweises auf die Patenterteilung: **21.04.2010**
(21) Anmeldenummer: 08007694.6
(22) Anmeldetag: 21.04.2008
(51) Int. Cl.: A61F 2/46

(54) **Chirurgisches Instrument zum Ausmessen eines Zwischenwirbelraumes**
Surgical instrument for measuring the space between vertebrae
Instrument chirurgical de mesure d'un espace intervertébrale

(43) Veröffentlichungstag der Anmeldung: 28.10.2009
(73) Patentinhaber: Weber, Helmut, 78576 Emmingen-Liptingen (DE)
(72) Erfinder: Weber, Helmut, 78576 Emmingen-Liptingen (DE)
(74) Vertreter: Modrow, Stephanie

(56) Entgegenhaltungen:
- WO-A-03/037230
- WO-A-2005/006944
- WO-A-2006/027098
- WO-A-2008/021972
- US-A1- 2006 074 431
- US-A1- 2007 260 260

## Beschreibung

Die Erfindung betrifft ein chirurgisches Instrument zum Ausmessen eines Zwischenwirbelraumes.

In der Bandscheibenprothetik ist es üblich, sogenannte Zwischenwirbelprothesen als Bandscheibenersatz zu verwenden. Derartige Zwischenwirbelprothesen werden im Rahmen einer Operation zwischen die Wirbelkörper eingesetzt und ersetzen die zuvor entfernte defekte Bandscheibe. Um eine möglichst gute Wiederherstellung der ursprünglichen Beweglichkeit und Funktionalität der Wirbelsäule zu gewährleisten, ist es notwendig, dass der geschädigte Zwischenwirbelraum möglichst genau rekonstruiert wird und die Abmessungen und Positionierung einer Zwischenwirbelprothese optimal ausgewählt werden. Zwischenwirbelprothesen der angesprochenen Art bestehen in der Regel aus zwei Prothesenplatten, welche jeweils mit einem Wirbelkörper verbunden werden. In einigen Varianten ist zwischen den Prothesenplatten ein Prothesenkern angeordnet, auf dem die Prothesenplatten gleiten und der die dämpfende Funktion der entfernten Bandscheibe übernimmt. Bei der Bestimmung der Abmessungen für eine einzusetzende Zwischenwirbelprothese sind insbesondere die Fläche der Prothesenplatten, eine Gesamthöhe der Zwischenwirbelprothese sowie eine Winkelstellung der den Wirbelkörpern zugewandten Oberflächen der Prothesenplatten zueinander ausschlaggebend.

Gemäß dem Stand der Technik werden die Maße der einzusetzenden Zwischenwirbelprothese präoperativ anhand von Röntgen- und/oder CT-Aufnahmen ermittelt und wird während der Operation nach dem Ausräumen des Bandscheibenfaches durch probeweises Einsetzen von Implantaten verifiziert. Die Verifikation der Implantatmaße erfolgt dabei derart, dass ein Probeimplantat zwischen die mechanisch auseinander gespreizten Wirbelkörper eingesetzt wird, die Spreizung gelöst wird und anschließend anhand einer Röntgenaufnahme die Passform des Implantats überprüft wird.

Nachteilhaft an dieser bisherigen Operationsmethode ist vor allem, dass zum Einsetzen und Überprüfen von Zwischenwirbelprothesen verschiedener Abmessungen ein mehrfaches Spreizen der Wirbelkörper zum Herausnehmen und Wiedereinsetzen der Testprothesen notwendig ist. Dies nimmt einerseits einen großen Teil der Operationszeit in Anspruch und bringt andererseits unnötige mechanische Belastungen der Wirbelsäule des Patienten mit sich.

Als Stand der Technik werden die US 2006/074431 A1,
WO 2008/021972 A, WO 03/037230 A, WO 2006/027098 A,
US 2007/260260 A1 und WO 2005/006944 A genannt.

Die US 2006/074431 stellt den nächstliegenden Stand der Technik das. Aufgabe der Erfindung ist es, ein chirurgisches Instrument zum Ausmessen eines Zwischenwirbelraumes zur Verfügung zu stellen, mit dem die Bestimmung der Abmessungen für eine einzusetzende Zwischenwirbelprothese schneller, einfacher und schonender für den Patienten möglich ist.

Diese Aufgabe wird durch ein chirurgisches Instrument mit den Merkmalen des Patentanspruchs 1 gelöst.

Vorteilhafte Weiterbildungen sind Gegenstand der Unteransprüche.

Ein erfindungsgemäßes chirurgisches Instrument zum Ausmessen eines Zwischenwirbelraumes weist an einem distalen Ende eines Griffelements wenigstens zwei Messplatten auf, wobei ein Abstand der Messplatten und ein von den Messplatten eingeschlossener Winkel einstellbar sind. Der Vorteil des erfindungsgemäßen chirurgischen Instruments liegt darin, dass durch die Einstellbarkeit von Abstand und eingeschlossenem Winkel der Messplatten nur ein Spreizvorgang für das Einsetzen des chirurgischen Instruments notwendig ist, mit diesem durch die Einstellbarkeit des Abstands und des eingeschlossnen Winkels der Messplatten eine in situ Variation der Größen möglich ist, so dass durch ein einmaliges Einsetzen des chirurgischen Instruments verschiedene Implantatabmessungen auf ihre Passgenauigkeit überprüft werden können und anschließend direkt eine Zwischenwirbelprothese mit den korrekten Abmessungen eingesetzt werden kann.

Es ist vorteilhaft, wenn gleichzeitig mit den Implantatabmessungen auch eine Einbautiefe für das Implantat ausgehend von der anterioren Seite des Wirbelkörpers über einen bewegbaren Tiefenanschlag bestimmbar ist. Ein derartiger Tiefenanschlag kann beispielsweise über eine das Griffelement umgreifende Hülse bestimmt werden, welche beispielsweise durch eine über ein Außengewinde schraubbare zylindrische Hülse realisiert werden kann. Der Vorteil einer zylindrischen Hülse, die das gesamte Griffelement umgreift, ist, dass lediglich ein einziger Anschlag vorhanden ist und somit mit einer einzelnen Einstellung der Anschlag gleichzeitig für beide Wirbelkörper, zwischen denen die Zwischenwirbelprothese montiert werden soll, bestimmt werden kann.

Für eine mechanisch einfache Ausführung des chirurgischen Instruments ist es vorteilhaft, wenn eine erste Messplatte zur Einstellung des Abstandes und eine zweite Messplatte zur Einstellung des zwischen den Messplatten eingeschlossenen Winkels ausgebildet ist. Die mechanische Ausführung ist in diesem Fall besonders einfach, so dass für eine Platte lediglich eine Mechanik zur Höhenverstellung und für die andere Platte lediglich eine Mechanik zur Verstellung des Plattenwinkels vorgesehen sein muss.

Eine einfache Höhenverstellung ist erfindungsgemäß mit einer Spreizvorrichtung, die nach dem Prinzip eines Scherenhubtisches ausgebildet ist, zu bewerkstelligen. Dabei ist es vorteilhaft, dass durch eine solche Spreizvorrichtung eine parallele Verstellung der Messplatte erfolgt und kein zusätzliches Kippen auftritt.

Eine derartige Spreizvorrichtung weist erfindungsgemäß zwei gegeneinander bewegbare Scherenglieder auf, von denen eines mittels einer Kuppelstange angesteuert wird, wobei das andere mittels einer Achse drehbar gelagert ist.

Eine lineare Bewegung der Kuppelstange kann aus einer rotatorischen Bewegung einer Gewindestange erfindungsgemäß besonders einfach durch eine Gewindebuchse, die drehfest in dem Griffelement gelagert ist, erzeugt werden. Durch eine derartige Ansteuerung ist es besonders einfach möglich, kleine Bewegungsschritte des einen Scherengliedes durch ein Gewinde mit einer geringen Ganghöhe präzise einzustellen. Dies kann beispielsweise durch eine Gewindestange, die in dem Griffelement angeordnet ist, erfolgen. An einem proximalen Ende einer solchen Gewindestange kann dann beispielsweise ein Bedienhebel oder ein entsprechend ausgebildeter Drehknopf angeordnet sein. Der Vorteil der Bedienung über eine Gewindestange liegt darin, dass über die Wahl des Gewindes besonders einfach eine Untersetzung für den Einstellweg erreicht werden kann.

Zur Einstellung des von der Messplatte eingeschlossenen Winkels ist erfindungsgemäß eine Schwenkvorrichtung vorgesehen. Eine derartige Schwenkvorrichtung kann besonders einfach derart gebildet werden, dass die zweite Messplatte entlang einer senkrecht zur Längsachse des Griffelementes und parallel zu der ersten Messplatte angeordneten Achse schwenkbar gelagert ist. Es ist besonders vorteilhaft, wenn diese Achse in einem zentralen Bereich der zweiten Messplatte vorgesehen ist, so dass sich durch ein Schwenken der zweiten Messplatte der zentral ermittelte Abstand der Messplatten nicht verändert.

Eine Schwenkbewegung der zweiten Messplatte ist erfindungsgemäß besonders einfach durch eine Pleuelstange aus einer linearen Bewegung erzeugbar. Eine solche Pleuelstange wandelt eine lineare Bewegung, beispielsweise die einer zweiten Gewindebuchse, in eine rotatorische Bewegung, also die Schwenkbewegung der zweiten Messplatte, um.

Die lineare Bewegung der zweiten Gewindebuchse ist erfindungsgemäß wiederum besonders einfach über eine zweite Gewindestange, die ebenfalls in dem Griffelement gelagert sein kann, einstellbar. Eine besonders elegante Lösung ergibt sich, wenn das Griffelement rohrförmig ausgebildet ist und die zweite Gewindestange in der ersten Gewindestange, die ebenfalls als Rohr ausgebildet ist, gelagert ist.

Diese Lösung ist für die Gewindebuchse ebenfalls besonders e-legant. Erfindungsgemäß ist die zweite Gewindebuchse konzentrisch zu der ersten Gewindebuchse angeordnet und in dieser gleitend gelagert.

Das gesamte chirurgische Instrument ist vorteilhafterweise aus biokompatiblem und sterilisierbarem Material, z. B. rostfreier Stahl oder Titan, gefertigt. Der Handgriff kann beispielsweise mit einem Silikongummi umspritzt sein, um eine bessere Handhabbarkeit zu gewährleisten.

Die Erfindung wird nachfolgend anhand eines Ausführungsbeispiels unter Bezugnahme auf die beigefügten Figuren näher beschrieben.

### Es zeigen:

- Figur 1a:: Eine Draufsicht auf ein erfindungsgemäßes chirurgisches Instrument,
- Figur 1b:: eine Seitenansicht des chirurgischen Instrumentes aus Figur 1a,
- Figur 2:: eine seitliche Schnittdarstellung des distalen Endes des chirurgischen Instrumentes aus Figur 1b,
- Figur 3a:: eine erste Gewindebuchse in Seitenansicht,
- Figur 3b:: die Gewindebuchse aus Figur 3a in Draufsicht,
- Figur 3c:: die Gewindebuchse aus den Figuren 3a und 3b in Vorderansicht,
- Figur 4a:: eine zweite Gewindebuchse in Seitenansicht,
- Figur 4b:: die Gewindebuchse aus Figur 4a in Draufsicht,
- Figur 4c:: die Gewindebuchse aus den Figuren 4a und 4b in Vorderansicht.

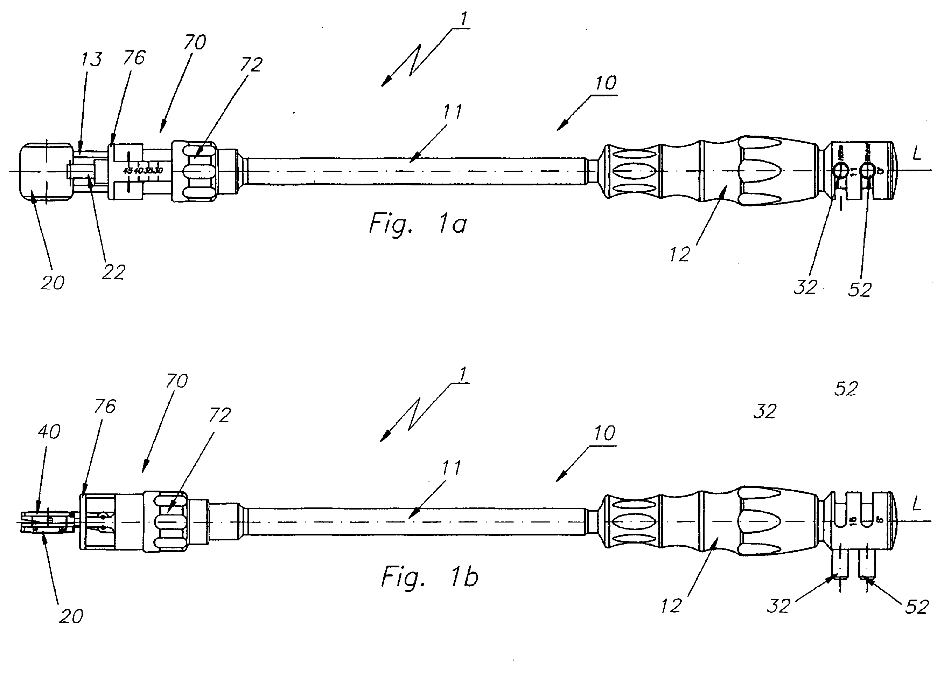

Figur 1a zeigt eine Draufsicht auf ein erfindungsgemäßes chirurgisches Instrument 1 zum Ausmessen eines Zwischenwirbelraumes. Das chirurgische Instrument 1 weist ein Griffelement 10 auf, das aus einem Handgriff 12 sowie einem an dem Handgriff 12 angeordneten Schaft 11 besteht. Am distalen Ende des Griffelements 11 ist eine Plattenhalterung 13 angeordnet, an der zwei Messplatten 20, 40 angeordnet sind. An dem distalen Ende des Griffelements 10 ist von distal gesehen hinter den Messplatten 20, 40 des Weiteren ein beweglicher und einstellbarer Tiefenanschlag 70 angeordnet, der im Wesentlichen eine drehbare Hülse 72, die auf einem Außengewinde 74 des Griffelements 11 sitzt, sowie einen mit dieser Hülse verbundenen Anschlag 76 aufweist. Der Anschlag 76 umgreift das chirurgische Instrument 1 im distalen Bereich vollständig, so dass mittels des Anschlags 76 gleichzeitig für einen oberhalb und einen unterhalb der Messplatten 20, 40 befindlichen Wirbelkörper ein Tiefenanschlag einstellbar ist.

Am proximalen Ende des Griffelements 10 ist hinter dem Handgriff 12 eine Betätigungseinrichtung mit einem ersten Betätigungshebel 32 und einem zweiten Betätigungshebel 52 angeordnet. Die Betätigungshebel 32, 52 wirken über in dem Griffelement 10 angeordnete Gewindestangen auf die im distalen Bereich angeordnete Einstellmechanik für die Messplatten 20, 40.

In Figur 1b ist das chirurgische Instrument aus Figur 1a in Seitenansicht dargestellt. In dieser Seitenansicht sind am proximalen Ende des Griffelements 10 besonders gut die Betätigungshebel 32, 52 zu erkennen, die radial an den im Griffelement 10 angeordneten Gewindestangen angeordnet sind.

Am distalen Ende des Griffelements 10 ist der Tiefenanschlag 70 bis in eine distale Endstellung gedreht, d. h. dass der Anschlag 76 unmittelbar hinter den Messplatten 20, 40 liegt, während er in Figur 1a in einer proximalen Endposition direkt am Übergang des Griffelements 10 zu der Plattenhalterung 13 sitzt.

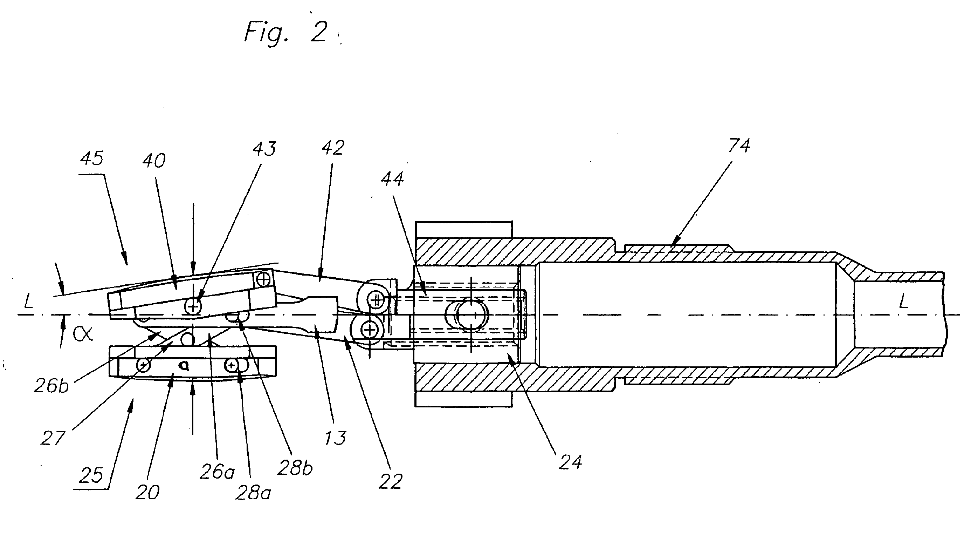

Figur 2 zeigt die Einstellmechanik der distal angeordneten Messplatten 20, 40 aus den Figuren 1a und 1b in einer vergrößerten Schnittdarstellung. Für eine verbesserte Übersichtlichkeit ist der Tiefenanschlag 70 in dieser Darstellung nicht abgebildet.

In der Schnittdarstellung aus Figur 2 ist im rechten Bildabschnitt der distale Bereich des Griffelements 10 abgebildet, an den sich die Plattenhalterung 13 anschließt. An der Plattenhalterung 13 ist über eine Scherenmechanik 27, welche insbesondere nach dem Prinzip eines Scherenhubtisches bewegbar ist, eine erste Messplatte 20, deren Abstand relativ zu der Plattenhalterung 13 und der zweiten Messplatte 40 verstellbar ist, angeordnet. An der Plattenhalterung 13 ist weiterhin, gelagert über eine Achse 43, die zweite Messplatte 40 angeordnet, welche zumindest in einer Position parallel zur ersten Messplatte 20 angeordnet ist. Weiterhin ist der Neigungswinkel α der zweiten Messplatte 40 relativ zu der ersten Messplatte 20 über eine Pleuelstange 42 veränderbar.

Die Scherenmechanik 27 der ersten Messplatte 20 ist über eine Kuppelstange 22 linear ansteuerbar, d. h. eine lineare Bewegung einer Gewindebuchse 24 wird über die Kuppelstange 22 auf ein erstes Scherenglied 26a der Scherenmechanik 27 übertragen. Die Scherenglieder 26a, 26b der Scherenmechanik 27 sind drehbar und gegeneinander bewegbar miteinander verbunden. Ein erstes Ende des ersten Scherengliedes 26a ist in einem ersten Langloch 28a in der Plattenhalterung 13 drehbar und entlang der Längsachse L verschiebbar gelagert. Ein zweites Ende des ersten Scherengliedes 26a ist über eine Achse in der ersten Messplatte 20 gelagert. Ein erstes Ende des zweiten Scherengliedes 26b ist über eine Achse drehbar in der Plattenhalterung 13 gelagert, während ein zweites Ende des zweiten Scherengliedes 26b in einem zweiten Langloch 28b in der ersten Messplatte 20 drehbar und parallel zu der Längsachse L verschiebbar gelagert ist. Die Kuppelstange 22 wirkt auf das in dem ersten Langloch 28a der Plattenhalterung 13 gelagerte erste Ende des ersten Scherengliedes 26a. Durch eine Verschiebung des ersten Scherengliedes 26a entlang der Längsachse L des Griffelements 10 ändert sich nach dem Prinzip des Scherenhubtisches der Abstand der ersten Messplatte 20 zur Plattenhalterung 13 und damit der Abstand a der Messplatten 20, 40, der zwischen den Oberflächen der Messplatten 20, 40 normal zur ersten Messplatte 20 an der Stelle der Achse 43 definiert ist.

Die zweite Messplatte 40 ist über eine zentral angeordnete Achse 43 an der Plattenhalterung 13 gelagert. Über eine Pleuelstange 42, die an dem proximalen Ende der zweiten Messplatte 40 angreift ist eine lineare Bewegung einer zweiten Gewindebuchse 44 in eine Kippbewegung der zweiten Messplatte 20 um die Achse 43 überführbar. Die Lagerung der zweiten Messplatte 40 erfolgt derart, dass durch eine Kippbewegung der zweiten Messplatte 40 in einem zentralen Bereich der Messplatten 20, 40 keine Abstandsänderung erzeugt wird.

Die Ansteuerung der Kuppelstange 22 sowie der Pleuelstange 42 erfolgt über die in dem Griffelement gelagerten Gewindebuchsen 24, 44. Die erste Gewindebuchse 24 ist in dem Griffelement 10 drehfest gelagert und als Hülse 241 ausgebildet, in der die zweite Gewindebuchse 44 sitzt. Die erste Gewindebuchse 24 wird über eine erste rohrförmig ausgebildete Gewindestange angesteuert und übersetzt eine Drehbewegung der ersten Gewindestange 31 in eine lineare Bewegung entlang der Längsachse L der ersten Gewindebuchse 24, so dass durch Drehen der ersten Gewindestange die Höhe der ersten Messplatte 20 verstellbar ist. In der ersten rohrförmig ausgebildeten Gewindestange sitzt eine zweite Gewindestange, die die zweite Gewindebuchse 44 ansteuert. Die zweite Gewindebuchse 44 ist über eine Anlagefläche 449 drehfest in der ersten Gewindebuchse 25 gelagert.

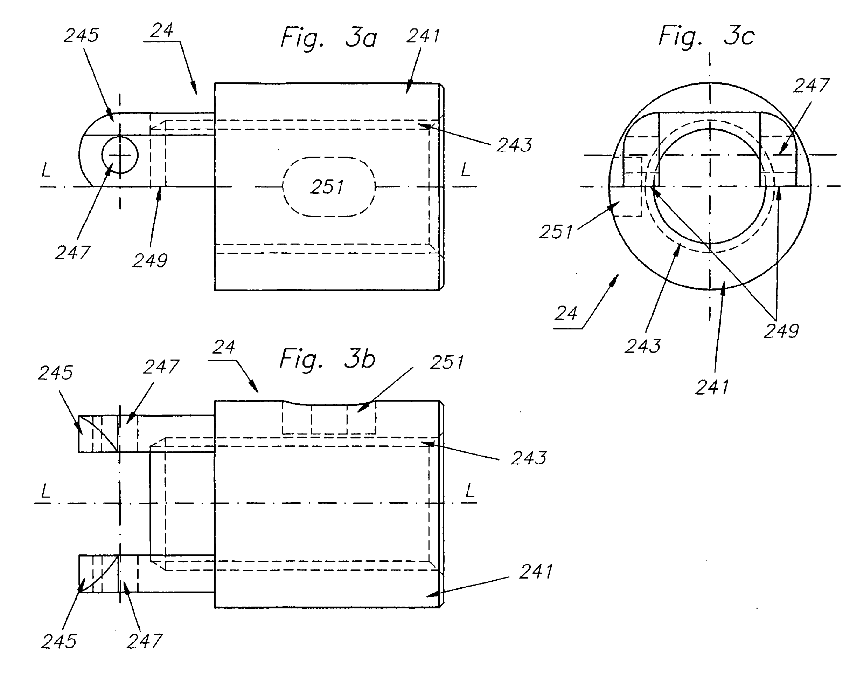

Die Figuren 3a bis 3c zeigen verschieden Ansichten der ersten Gewindebuchse 24, wie sie in Figur 2 zur Ansteuerung der ersten Messplatte 20 verwendet wird.

In Figur 3a ist eine Seitenansicht der ersten Gewindebuchse 24 dargestellt, in der besonders gut der Aufbau der ersten Gewindebuchse 24 erkennbar ist. Die erste Gewindebuchse 24 besteht im Wesentlichen aus einer Hülse 241, in die ein Innengewinde 243 eingebracht ist. Vorderseitig ist an der Hülse 241 ein gabelförmiger Fortsatz 245 mit jeweils einer Bohrung 247 in beiden Schenkeln angeordnet, wobei über die Bohrung 247 die Verbindung zu der Kuppelstange 22 herstellbar ist. In das Innengewinde 243 greift ein Außengewinde der ersten Gewindestange ein. Die erste Gewindebuchse 24 ist über einen Bolzen, der in ein am Umfang der Hülse 241 angeordnetes Langloch 251 eingreift, und in eine entsprechende Ausnehmung des Griffelements 10 sitzt drehfest in dem Griffelement 10 gelagert, so dass eine Rotation der ersten Gewindestange aufgrund des Gewindeeingriffs eine lineare Bewegung der ersten Gewindebuchse 24 hervorruft.

In Figur 3b ist besonders gut der gabelförmige Fortsatz 245 zu erkennen. Der gabelförmige Fortsatz 245 erstreckt sich außerhalb des Gewindedurchmessers von der Buchse 241 weg und bietet durch seine gabelförmige Ausführung und die Bohrungen 247 die Möglichkeit, die Kuppelstange 22 durch eine Achslagerung mit einer Achse quer zur Längsachse L an der Gewindebuchse 24 zu befestigen.

In der Vorderansicht von Figur 3c ist deutlich zu erkennen, dass sich der Fortsatz 245 nur in einer Hälfte der zylindrisch ausgeformten Gewindebuchse 24 erstreckt und zur Mittenachse gewandt eine Anlagefläche 249 ausbildet. Die Anlagefläche 249 steht im zusammengebauten Zustand in Kontakt mit einer Anlagefläche 449 der zweiten Gewindebuchse 44 und gewährleistet so deren drehfeste Lagerung.

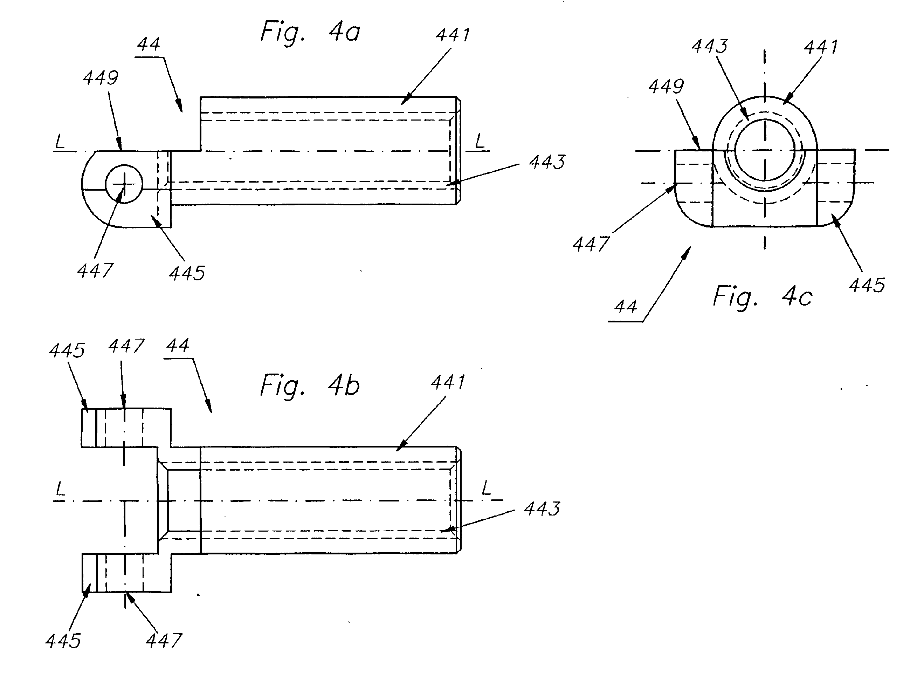

In den Figuren 4a bis 4c sind verschiedene Ansichten der zweiten Gewindebuchse 44, wie sie gemäß Figur 2 für die Ansteuerung der Schwenkvorrichtung 45 verwendet wird, dargestellt.

In Figur 4a ist eine Seitenansicht der zweiten Gewindebuchse 44 gezeigt. In dieser Ansicht ist besonders gut der Aufbau der zweiten Gewindebuchse 44 erkennbar. Die zweite Gewindebuchse 44 besteht im Wesentlichen aus einer Hülse 441, in die ein Innengewinde 443 eingebracht ist. Vorderseitig erstreckt sich von der Hülse 441 ausgehend ein gabelförmiger Fortsatz 445, in dem eine Bohrung 447 angeordnet ist. Über die Bohrung 447 kann die Verbindung zu der Pleuelstange 42 hergestellt werden. Wie auch bei der ersten Gewindebuchse 24 ist der Fortsatz 445 nur bis zur Hälfte des Radius der Hülse 441 ausgebildet und bildet dort eine Anlagefläche 449, die in zusammengebautem Zustand an der Anlagefläche 249 der ersten Gewindebuchse 24 anliegt. Durch die formschlüssige Anordnung dieser Anlageflächen 249, 449 ist eine drehfeste Lagerung der zweiten Gewindebuchse 44 in der ersten Gewindebuchse 24 gewährleistet.

Figur 4b zeigt eine Draufsicht auf die zweite Gewindebuchse 44. In dieser Draufsicht ist besonders gut der gabelförmige Fortsatz 445 mit der in beiden Schenkeln des Fortsatzes 445 eingebrachten Bohrung 447 zu erkennen. Durch die Bohrungen 447 ist in zusammengebautem Zustand wiederum eine Achse quer zur Längsachse L geführt, mittels derer die Pleuelstange 42 gelagert ist, so dass eine lineare Bewegung der Gewindebuchse 44 über die Pleuelstange 42 auf die zweite Messplatte 40 übertragbar ist.

Figur 4c zeigt eine Vorderansicht der zweiten Gewindebuchse 44, in der nochmals besonders gut die durch den Fortsatz 445 gebildete Anlagefläche 449 zu erkennen ist.

Eine Betätigung der beiden Messplatten 20, 40 erfolgt gemäß dem oben vorgestellten Aufbau durch eine rotatorische Betätigung eines der beiden Betätigungshebel 32, 52 um die Längsachse des Griffelements 10. Durch eine Betätigung des ersten Betätigungshebels 32 wird eine Drehung der rohrförmig ausgebildeten ersten Gewindestange bewirkt, was zu einer translatorischen Bewegung der ersten Gewindebuchse 24 am distalen Ende des Griffelements 10 führt. Durch die translatorische Bewegung der ersten Gewindebuchse 24 wird über die Kuppelstange 22 eines der Scherenglieder 26 derart bewegt, dass über die Scherenmechanik 27 der Plattenabstand a zwischen den Messplatten 20, 40 verändert wird.

Bei einer Betätigung des zweiten Betätigungshebels 52 wird eine rotatorische Bewegung der zweiten Gewindestange, die in der ersten Gewindestange gelagert ist, bewirkt. Durch diese rotatorische Bewegung wird eine translatorische Bewegung der zweiten Gewindebuchse 44 bewirkt, die wiederum über die Pleuelstange 42 eine Kippbewegung der zweiten Messplatte 40 um die Achse 43 einleitet. Auf diese Art wird der von den Messplatten 20, 40 eingeschlossene Winkel α einstellbar.

Bei der Operation werden also nach dem Aufspreizen und Ausräumen des Bandscheibenfaches die Messplatten 20, 40 des chirurgischen Instrumentes 1 in den Zwischenwirbelraumes eingeführt, wobei eine erste Grundeinstellung entsprechend der durch Röntgen oder Computertomografie ermittelten Werte erfolgt. Anschließend wird in einer weiteren Röntgenaufnahme die Passgenauigkeit der Messplatten 20, 40 des chirurgischen Instrumentes 1, also auch die Passgenauigkeit einer mit den entsprechenden Werten bereitgestellten Prothese überprüft. Falls nicht die gewünschte Passgenauigkeit erreicht wird, ist es mit dem erfindungsgemäßen chirurgischen Instrument 1 möglich, in situ eine Maßanpassung vorzunehmen und in einer weiteren Röntgenaufnahme eine weitere Passgenauigkeits-Kontrolle durchzuführen. Ein erneutes Spreizen der Wirbel zum Entnehmen und erneuten Einsetzen eines Probeimplantats ist nicht notwendig. Auf diese Weise wird eine wesentliche Zeitersparnis erreicht und insbesondere für den Patienten eine schonendere Operation ermöglicht.

### Bezugszeichenliste

- 1: Chirurgisches Instrument
- 10: Griffelement
- 11: Schaft
- 12: Handgriff
- 13: Plattenhalterung
- 20: erste Messplatte
- 22: Kuppelstange
- 24: erste Gewindebuchse
- 25: Spreizvorrichtung
- 26a: erstes Scherenglied
- 26b: zweites Scherenglied
- 27: Scherenmechanik
- 28a: erstes Langloch
- 28b: zweites Langloch
- 29: Verbindungslager
- 32: erster Betätigungshebel
- 40: zweite Messplatte
- 42: Pleuelstange
- 43: Achse
- 44: zweite Gewindebuchse
- 45: Schwenkvorrichtung
- 49: Lager
- 52: zweiter Betätigungshebel
- 70: Tiefenanschlag
- 72: Hülse
- 74: Außengewinde
- 76: Anschlag

- a: Abstand
- α: Winkel
- L: Längsachse

- 241: Hülse
- 243: Innengewinde
- 245: Fortsatz
- 247: Bohrung
- 249: Anlagefläche
- 251: Langloch

- 441: Hülse
- 443: Innengewinde
- 445: Fortsatz
- 447: Anlagefläche
- 449: Anlagefläche

## Patentansprüche

1. Chirurgisches Instrument (1) zum Ausmessen eines Zwischenwirbelraumes, das an einem distalen Ende eines Griffelements (10) wenigstens eine erste und eine zweite Messplatte (20, 40) aufweist, wobei ein Abstand (a) der Messplatten (20, 40) und ein von den Messplatten (20, 40) eingeschlossener Winkel (α) einstellbar sind,
**dadurch gekennzeichnet, dass**
zur Einstellung des Abstand (a) der Messplatten (20, 40) eine Spreizvorrichtung (25) vorgesehen ist, welche nach dem Prinzip eines Scherenhubtisches ausgebildet ist, welcher zwei gegeneinander bewegbare Scherenglieder (26) aufweist, wobei eine Kuppelstange (22) zur Ansteuerung eines der Scherenglieder (26) vorgesehen ist, wobei eine erste Gewindebuchse (24) zur Erzeugung einer linearen Bewegung der Kuppelstange (22) aus einer rotatorischen Bewegung einer ersten Gewindestange, die in dem Griffelement (10) angeordnet ist, vorgesehen ist, und dass eine Schwenkvorrichtung (45) zur Einstellung des von den Messplatten (20, 40) eingeschlossenen Winkels (α) vorgesehen ist, wobei eine Pleuelstange (42) zur Erzeugung einer Schwenkbewegung der zweiten Messplatte (40) aus einer linearen Bewegung vorgesehen ist, wobei eine zweite Gewindebuchse (44) zur Erzeugung der linearen Bewegung der Pleuelstange (42) aus einer rotatorischen Bewegung einer zweiten Gewindestange, die in dem Griffelement (10) angeordnet ist, vorgesehen ist und wobei die zweite Gewindebuchse (44) konzentrisch zu der ersten Gewindebuchse (24) angeordnet und in dieser gleitend gelagert ist.

2. Chirurgisches Instrument (1) nach Anspruch 1,
**dadurch gekennzeichnet, dass**
am distalen Ende des Griffelements (10) ein entlang einer Längsachse (L) des Griffelements bewegbarer Tiefenanschlag (70) vorgesehen ist.

3. Chirurgisches Instrument (1) nach einem der vorhergehenden Ansprüche,
**dadurch gekennzeichnet, dass**
die erste Messplatte (20) zur Einstellung des Abstands (a) und die zweite Messplatte (40) zur Einstellung des eingeschlossenen Winkels (α) ausgebildet ist.

4. Chirurgisches Instrument (1) nach einem der vorhergehenden Ansprüche,
**dadurch gekennzeichnet, dass**
zur Bildung der Schwenkvorrichtung (45) die zweite Messplatte (40) entlang einer senkrecht zur Längsachse (L) des Griffelementes (10) und parallel zu der ersten Messplatte (20) angeordneten Achse (43) schwenkbar gelagert ist.

5. Chirurgisches Instrument (1) nach einem der vorhergehenden Ansprüche,
**dadurch gekennzeichnet, dass**
die erste Gewindestange als Rohr ausgebildet ist und die zweite Gewindestange konzentrisch in dieser angeordnet ist.

6. Chirurgisches Instrument (1) nach einem der vorhergehenden Ansprüche,
**dadurch gekennzeichnet, dass**
das chirurgische Instrument (1) aus biokompatiblem Material, insbesondere rostfreien Stählen oder Titan, gefertigt ist.

## Claims

1. Surgical instrument (1) for measuring an intervertebral space, which comprises at least a first and a second measuring plate (20, 40) at a distal end of a handle element (10), wherein a spacing (a) of the measuring plates (20, 40) and an angle (α) enclosed by the measuring plates (20, 40) are adjustable, **characterised in that**, in order to adjust the spacing (a) of the measuring plates (20, 40), a spreading device (25) is provided which is designed according to the principle of a scissors-type lifting table, which has two scissor members (26) that can be moved relative to one another, wherein a coupling rod (22) is provided for actuating one of the scissor members (26), wherein a first threaded bushing (24) is provided for generating a linear movement of the coupling rod (22) from a rotational movement of a first threaded rod which is arranged in the handle element (10), and **in that** a pivoting device (45) is provided for adjusting the angle (α) enclosed by the measuring plates (20, 40), wherein a connection rod (42) is provided for generating a pivoting movement of the second measuring plate (40) from a linear movement, wherein a second threaded bushing (44) is provided for generating the linear movement of the connection rod (42) from a rotational movement of a second threaded rod which is arranged in the handle element (10), and wherein the second threaded bushing (44) is arranged concentric to the first threaded bushing (24) and is mounted in a sliding manner therein.

2. Surgical instrument (1) according to claim 1,
**characterised in that** a depth stop (70) movable along a longitudinal axis (L) of the handle element is provided at the distal end of the handle element (10).

3. Surgical instrument (1) according to one of the preceding claims, **characterised in that** the first measuring plate (20) is designed to adjust the spacing (a) and the second measuring plate (40) is designed to adjust the enclosed angle (α).

4. Surgical instrument (1) according to one of the preceding claims, **characterised in that**, in order to form the pivoting device (45), the second measuring plate (40) is mounted such that it can pivot about an axis (43) arranged perpendicular to the longitudinal axis (L) of the handle element (10) and parallel to the first measuring plate (20).

5. Surgical instrument (1) according to one of the preceding claims, **characterised in that** the first threaded rod is designed as a tube and the second threaded rod is arranged concentrically therein.

6. Surgical instrument (1) according to one of the preceding claims, **characterised in that** the surgical instrument (1) is made from biocompatible material, in particular stainless steels or titanium.

## Revendications

1. Instrument chirurgical (1) pour mesurer un espace intervertébral ayant à une extrémité distale d'un élément de préhension (10), au moins une première et une seconde plaque de mesure (20, 40), la distance (a) entre les plaques de mesure (20, 40) et l'angle (α) formé entre les plaques de mesure (20, 40) étant réglables,
**caractérisé en ce que**
un dispositif d'écartement (25) réalisé selon le principe d'une table relevable à leviers croisés, ayant deux organes en forme de ciseaux (26) mobiles l'un par rapport à l'autre, règle la distance (a) des plaques de mesure (20, 40),
une bielle (22) commande l'un des organes de cisaille (26),
un premier manchon fileté (24) génère un mouvement linéaire de la bielle (22) à partir du mouvement de rotation d'une première tige filetée associée à l'élément de préhension (10), et
un dispositif de basculement (45) règle l'angle (α) compris entre les plaques de mesure (20, 40),
une bielle (42) génère un mouvement de basculement de la seconde plaque de mesure (40) à partir d'un mouvement linéaire,
un second manchon fileté (44) génère le déplacement linéaire de la bielle (42) à partir d'un mouvement de rotation d'une seconde tige filetée associée à l'élément de préhension (10), et
le second manchon fileté (44) est concentrique au premier manchon fileté (24) dans lequel il est logé de manière coulissante.

2. Instrument chirurgical (1) selon la revendication 1,
**caractérisé en ce que**
l'extrémité distale de l'élément de préhension (10) comporte une unité de profondeur mobile (70) le long de l'axe longitudinal (L) de l'élément de préhension.

3. Instrument chirurgical (1) selon l'une des revendications précédentes,
**caractérisé en ce que**
la première plaque de mesure (20) est prévue pour régler la distance (a), la seconde plaque de mesure (40) réglant l'angle (α).

4. Instrument chirurgical (1) selon l'une des revendications précédentes,
**caractérisé en ce que**
pour former le dispositif de basculement (45), la seconde plaque de mesure (40) est montée basculante le long d'un axe (43) perpendiculaire à l'axe longitudinal (L) de l'élément de préhension (10) et parallèle à la première plaque de mesure (20).

5. Instrument chirurgical (1) selon l'une des revendications précédentes,
**caractérisé en ce que**
la première tige filetée est un tube et la seconde tige filetée est concentrique à celle-ci.

6. Instrument chirurgical (1) selon l'une des revendications précédentes,
**caractérisé en ce que**
l'instrument chirurgical (1) est en une matière biocompatible, notamment en des aciers inoxydables ou en titane.
